# EUROPEAN PATENT APPLICATION

(11) **EP 2 556 837 A1**
(43) Date of publication of application: **13.02.2013**
(21) Application number: 10735173.6
(22) Date of filing: 15.04.2010
(51) Int. Cl.: A61K 36/9064, A61K 36/898, A61K 36/86, A61K 36/72, A61K 36/718, A61K 36/67, A61K 36/53, A61K 36/46, A61K 36/21, A61K 36/185, A61P 3/10, A61K 129/00, A61K 131/00, A61K 125/00

(54) **TRADITIONAL CHINESE MEDICINE COMPOSITION FOR TREATING HYPERGLYCEMIA AND PREPARATION METHOD THEREOF**

(30) Priority: 09.04.2010 CN 201010143026
(71) Applicant: Tongling Weixin Investment & Consulting Co., Ltd, Tongling, Anhui 244000 (CN)
(72) Inventor: ZHANG, Baoyu, Anhui 244000 (CN)
(74) Representative: Larcher, Dominique
(86) International application number: PCT/CN2010/000505
(87) International publication number: WO 2011/123994

(57) **Abstract**

The present invention relates to a herbal composition for treating hyperglycemia, a method for preparing said herbal composition.

## Description

### Technical Field

The present invention relates to a novel herbal composition for the treating hyperglycemia. More particularly, the present invention relates to a novel herbal composition which is effective against pyloric ligation induced ulcer model and histamine induced ulcer model. The present invention also relates to a method for the preparation of the composition.

### Background Art

Hyperglycemia is a condition in which a person has a high blood sugar (glucose) level as a result of the body either not producing enough insulin, or because body cells do not properly respond to the insulin that is produced. Insulin is a hormone produced in the pancreas which enables body cells to absorb glucose, to turn into energy. If the body cells do not absorb the glucose, the glucose accumulates in the blood (hyperglycemia), leading to various potential medical complications.

As of 2005 at least 157 million people worldwide suffer from hyperglycemia, or 3.1% of the population.

### Summary of the Invention

The main object of the present invention is to provide a novel herbal composition for treating hyperglycemia.

Yet another object of the present invention is to provide a process for the preparation of the composition.

In doing so, the present invention provides a herbal composition for treating hyperglycemia, the said composition is consisting of 4-10% by wt. of an extract from Radix Coptidis and 4-11% by wt. of an extract from Radix Lamiophlomidis and 5-8 % by wt. of an extract from Radix Achyranthis, 5-11 % by wt. of an extract from Herba Violae mundae, 5-9% by wt. of an extract from Fructus Amomi Rotundus, 8-11 % by wt. of an extract from Punica grantum, 4-9% by wt. of an extract from Pseudobulbus Bletillae, 2-11 % by wt. of an extract from Fructus Rhamni, 8-12% by wt. of an extract from Piper longue and 2- 11% by wt. of an extract from Coxtex Eucommiae along with one or more pharmaceutically acceptable additives/carriers. The composition can be easily prepared by getting all the chinese herbes, adding solvent to extract them, and mixing the extracts with one or more pharmaceutically acceptable additives/carriers, without losing antidiabetic activity. Furthermore, there was no undesirable side effects in animals that had been treated with up to 2000 mg/kg p. o. doses of the extract. Accordingly, the anti-diabetic activity of the extract can be used to control hyperglycemia and particularly NIDDM.

In another embodiment of the present invention, the extract is an aqueous extract.

Suitable additives include, but are not limited to a group of nutrients comprising proteins, carbohydrates, sugar, talc, magnesium sterate, cellulose, calcium carbonate, starch-gelatin paste, and/or pharmaceutically acceptable carrier, excipient, diluent, solvent and other hypoglycemic agents.

In another embodiment of the present invention, wherein the effective amount of the inventive composition may be a dosage that ranges from about 1 to about 3000-mg/kg p. o. /day; wherein the dosage regime may be continued for about 2 to about 4 weeks. Preferably, the effective amount is a dose that ranges between about 200 to about 1000 mg/kg p. o./day over about 2-4 weeks of treatment.

The beneficial effects of the administered composition should last for about 8 hours once administered to the animal or mammal. Indeed, the inventive extract composition can be about 20 times more effective as compared to the prior product, without inducing undesirable side effects in that animal or mammal.

Thus, the present invention provides a herbal composition for treating hyperglycemia, the said composition is consisting of 4-10% by wt. of an extract from Radix Coptidis and 4-11% by wt. of an extract from Radix Lamiophlomidis and 5-8 % by wt. of an extract from Radix Achyranthis, 5-11% by wt. of an extract from Herba Violae mundae, 5- 9% by wt. of an extract from Fructus Amomi Rotundus, 8-11 % by wt. of an extract from Punica grantum, 4-9% by wt. of an extract from Pseudobulbus Bletillae, 2-11 % by wt. of an extract from Fructus Rhamni, 8-12% by wt. of an extract from Piper longue and 2- 11 % by wt. of an extract from Coxtex Eucommiae along with one or more pharmaceutically acceptable additives/carriers.

In an embodiment of the present invention, the extract is an aqueous extract.

In another embodiment of the present invention, the plant part of Radix Coptidis, Radix Lamiophlomidis and Radix Achyranthis is root.

In still another embodiment of the present invention, the plant part of Vitis vinifera, Fructus Amomi Rotundus, Fructus Rhamni and Fructus Quispualis is fruit.

In yet another embodiment of the present invention, the plant part of Pashica grantum is fruit rind.

In one more embodiment of the present invention, the plant part of Pseudobulbus Bletillae is rhizome.

In one another embodiment of the present invention, the plant part of Coxtex Eucommiae is bark.

In another embodiment of the present invention, the additives are selected from a group of nutrients comprising proteins, carbohydrates, sugar, talc, magnesium sterate, cellulose, calcium carbonate, starch-gelatin paste, and/or pharmaceutically acceptable carrier, excipient, diluent, solvent, and other hypoglycemic agents. Typically, the additives are not detrimental to the beneficial properties of the extract. In yet another embodiment of the present invention, the composition is administered orally as a capsule, tablet, syrup, concentrate, powder, granules, aerosol, or beads.

In accordance with the second object of the present invention, there is provided a process for the preparation of the novel herbal composition for treating hyperglycemia, the said process comprises getting all the chinese herbes, adding solvent to extract them, and mixing the extracts with one or more pharmaceutically acceptable additives/carriers.

In another embodiment of the present invention, the extract is an aqueous extract.

The invention is described in the examples given below which are provided by a way of illustrations only and should riot be construed to limit the scope of the present invention.

### EXAMPLES

### Example-1

The dose of streptozotocin is used to induce hyperglycemia mimicking to NIDDM, encountered clinically in Majority of patients. The NIDDM diabetic rats when treated with the composition according to the invention. for 2-3 weeks recovered to normal state, whereas the rats of control NIDDM diabetic group continued to have hyperglycemia and died in due course of time.

### Example-2

Get 9% by wt. of an extract from Radix Coptidis and 7% by wt. of an extract from Radix Lamiophlomidis and 6% by wt. of an extract from Radix Achyranthis, 8% by wt. of an extract from Herba Violae mundae, 6% by wt. of an extract from Fructus Amomi Rotundus, 9% by wt. of an extract from Punica grantum, 5% by wt. of an extract from Pseudobulbus Bletillae, 4% by wt. of an extract from Fructus Rhamni, 8% by wt. of an extract from Piper longue and 7% by wt. of an extract from Coxtex Eucommiae along with one or more pharmaceutically acceptable additives/carriers to make the composition according to the invention.

The composition (200mg/kg p. o.) showed significant hypoglycemic activity as tested on normal, 18h fasting, glucose loaded & streptozotocin induced hyperglycaemic rats. The onset of effect within 1/2h and the effect lasted for more than five hours. The fall of blood sugar recorded with above dose was 26mg/dl (n=24) as compared to fall of 17 & 23 mg/dl recorded with tolbutamide, 50mg/kg p. o. (n=9) and glipizide, 0. 5mg/kg p. o. (n=8) respectively in 18h fasting rats. <BR> <BR> <P>The hypoglycaemic activity was also recorded with plant powder (2. 5g/kg p. o.) and flavonoid glycoside isolated from the plant.

### Example-3

Get 6% by wt. of an extract from Radix Coptidis and 10% by wt. of an extract from Radix Lamiophlomidis and 5% by wt. of an extract from Radix Achyranthis, 10% by wt. of an extract from Herba Violae mundae, 5% by wt. of an extract from Fructus Amomi Rotundus, 8% by wt. of an extract from Punica grantum, 10% by wt. of an extract from Pseudobulbus Bletillae, 8% by wt. of an extract from Fructus Rhamni, 8% by wt. of an extract from Piper longue and 10% by wt. of an extract from Coxtex Eucommiae along with one or more pharmaceutically acceptable additives/carriers to make the composition according to the invention.

There was highly significant recovery of rats from <BR> <BR> non-insulin dependent hyperglycemia mellitus treated with the composition (200mg/kg p. o. /day) for 2- 3 weeks as per with following parameters : i) Blood glucose level, ii) Effect on body weight, iii) Survival of the animals, iv) Water intake, urine output & presence of glucose in urine, v) General condition of the animals and vi) Visit of ants to the voided urine of the rats.

### Example-4

Get 4% by wt. of an extract from Radix Coptidis and 5% by wt. of an extract from Radix Lamiophlomidis and 5% by wt. of an extract from Radix Achyranthis, 6% by wt. of an extract from Herba Violae mundae, 9% by wt. of an extract from Fructus Amomi Rotundus, 10% by wt. of an extract from Punica grantum, 4% by wt. of an extract from Pseudobulbus Bletillae, 9% by wt. of an extract from Fructus Rhamni, 10% by wt. of an extract from Piper longue and 5% by wt. of an extract from Coxtex Eucommiae along with one or more pharmaceutically acceptable additives/carriers to make the composition according to the invention.

There was highly significant recovery of rats form non-insulin dependent hyperglycemia mellitus treated with KA <BR> <BR> (200mglkg p. o. /day) for 2-3 weeks as per with following parameters: 1) Blood glucose level, ii) Effect on body weight, iii) Survival of the animals, iv) Water intake, urine output & presence of glucose in urine, v) General condition of the animals and vi) Visit of ants to the voided urine of the rats.

## Claims

1. A herbal composition for treating hyperglycemia, wherein the composition is consisting of 4-10% by wt. of an extract from Radix Coptidis and 4-11% by wt. of an extract from Radix Lamiophlomidis and 5-8 % by wt. of an extract from Radix Achyranthis, 5-11% by wt. of an extract from Herba Violae mundae, 5- 9% by wt. of an extract from Fructus Amorom Fructus Amomi Rotundus, 8-11 % by wt. of an extract from Punica grantum, 4-9% by wt. of an extract from Pseudobulbus Bletillae, 2-11 % by wt. of an extract from Fructus Rhamni, 8-12% by wt. of an extract from Piper longue and 2- 11 % by wt. of an extract from Coxtex Eucommiae along with one or more pharmaceutically acceptable additives/carriers.

2. A composition as claimed in claim 1, wherein the extract is an aqueous extract.

3. A process for the preparation of the herbal composition claimed in one of claim 1 or 2, wherein the said process comprises getting all the chinese herbes, adding solvent to extract them, and mixing the extracts with one or more pharmaceutically acceptable additives/carriers.

4. A process as claimed in claim 3, wherein the extract is an aqueous extract.
